(19) Europäisches Patentamt / European Patent Office / Office européen des brevets

(11) **EP 3 871 601 B1**

(12) **EUROPEAN PATENT SPECIFICATION**

(45) Date of publication and mention
of the grant of the patent:
**18.01.2023  Bulletin 2023/03**

(21) Application number: **19876541.4**

(22) Date of filing: **24.10.2019**

(51) International Patent Classification (IPC):
*A61B 5/12* *(2006.01)*          *H04R 3/04* *(2006.01)*
*H04R 29/00* *(2006.01)*      *H04R 25/00* *(2006.01)*

(52) Cooperative Patent Classification (CPC):
**A61B 5/121; H04R 3/04; H04R 29/001;**
A61B 2560/0223; H04R 25/70

(86) International application number:
**PCT/ES2019/070720**

(87) International publication number:
**WO 2020/084184 (30.04.2020 Gazette 2020/18)**

(54) **AUDIBILITY METER CALIBRATION SYSTEM**

KALIBRIERSYSTEM FÜR HÖRBARKEITSMESSGERÄT

SYSTÈME D'ÉTALONNAGE POUR UN AUDIOMÈTRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **25.10.2018  ES 201831036**

(43) Date of publication of application:
**01.09.2021  Bulletin 2021/35**

(73) Proprietor: **Devimetrix, S.L**
**08018 Barcelona (ES)**

(72) Inventor: **PLANA EGUIA, José María**
**08036 Barcelona (ES)**

(74) Representative: **Clarke Modet & Co.**
**C/ Suero de Quiñones 34-36**
**28002 Madrid (ES)**

(56) References cited:
EP-A1- 1 854 407          DE-A1- 4 038 211
US-A1- 2002 151 819      US-A1- 2007 261 491
US-A1- 2009 063 080      US-A1- 2011 009 770
US-A1- 2011 009 770

• Kiversal: "Audixi 10, the new digital
audiometer", , 21 August 2017 (2017-08-21),
pages 1-5, XP055823856, Retrieved from the
Internet:
URL:https://blog.kiversal.com/en/audixi-10
-the-new-digital-audiometer/ [retrieved on
2021-07-13]
• Kiversal: "New simplified calibration system", ,
11 February 2020 (2020-02-11), pages 1-6,
XP055823863, Retrieved from the Internet:
URL:https://trends.medicalexpo.com/kiversa
l/project-124801-432569.html [retrieved on
2021-07-13]
• Alan V Oppenheim , Alan Willsky: "Signals and
Systems", Prentice Hall signal processing series,
1997, pages 1-59, XP055808903,

## Description

### Technical field of the invention

The present invention belongs to the field of audiometry. In particular, it relates to techniques that can be applied for obtaining improved calibration of audibility meters and transducers associated with audiometric testing.

### Background of the invention

Audibility meters are used to measure and diagnose people's hearing ability. For measuring, very precise pure tones or other stimuli are presented to the patient by means of acoustic transducers (headphones, loudspeakers, vibrators). If the subject detects the tones or other stimuli, he indicates it by pressing a button or push-button. These pure tones, electronically generated by the audibility meter, are sent through phones (air conduction) or bone vibrators (bone conduction). The thresholds for different frequencies are measured in decibels. Frequencies in conventional audiometry for air conduction are usually between 125 Hz and 8 kHz or 16 kHz. For bone conduction they are usually between 250 Hz and 6 kHz.

The acoustic transducers connect directly to the audibility meter using standard stereo audio connectors (jacks) or to the wall of a soundproof booth which in turn connects to the audibility meter using the same stereo audio connectors.

In order to ensure correct measurements for clinical diagnosis, audibility meters and the transducers thereof must be regularly checked. This requires the transducers to be acoustically calibrated with the audibility meter to which they will be connected. Additionally, explicit care must be taken to properly carry out the connection, for example, in re-calibrations.

Electroacoustic simulation equipment of the human ear and head and sound level meters are used in the calibration. Generally, the entire audibility meter assembly and the transducers thereof are calibrated in a single electrical and acoustic phase. However, this type of calibration has a drawback. The transducers are only correctly calibrated with one audibility meter and therefore form a single calibrated assembly. Consequently, the exchange of transducers with other audibility meters causes a measurement error and, therefore, an unacceptable risk in clinical diagnosis.

Currently, calibration is addressed by taking the equipment to a specialised centre or having a technician travel to carry out the calibration on site. The acoustic transducers are associated with the audibility meter thereof with a label that further indicates the temporal validity of the calibration (expiration).

EP1854407A1 discloses an audiometer having an audiometer receiver and an audiometer body. The audiometer receiver includes a memory, a serial ROM, for storing identification and calibration information of the audiometer. It can detect misconnection of the audiometer receiver. It considers a calibration period of the audiometer receiver, and a correction amount of sound pressure required based on the information stored in the serial ROM.

US20110009770A1 discloses an audiometric testing device including a housing with integral calibration couplers to couple with a testing transducer. It also includes a tone generator to generate tones of various frequencies and intensities during a hearing test. A calibration transducer converts an output of the testing transducer into a calibration signal for a signal measurement module, which generates a calibration measurement to be used to correct for undesired intensity level variations produced by the tone generator.

Kiversal: "Audixi 10, the new digital audiometer", XP055823863, presents a commercial product, easy to use, with USB and internet connectivity. The product allows for automatic hearing tests and declares having an acoustic transducer and an audibility meter for instant metrological calibration.

### Brief description of the invention

In light of the limitations observed in the state of the art, there is therefore a need for an audibility meter calibration system that is capable of simplifying calibration and ensuring metrological veracity.

Specifically, a desirable aspect is to have the ability to connect any transducer already acoustically calibrated, and for the assembly of audibility meter with transducers to remain correctly calibrated. For a correct adaptation, the frequency response is read and self-adjusts to compensate if necessary.

Another additional aspect is that it recognises various types of transducers and ensures a correct connection.

Thanks to these and other functionalities, the management and maintenance of the individual calibration of both transducers and the audibility transducers thereof (headphones, bone vibrator, speakers) and the push-button for the patient.

FIG. 2A illustrates a block diagram of an embodiment of the system.
FIG. 2B illustrates another block diagram of an embodiment of the system.
FIG. 3 illustrates a schematic diagram of a transducer with a storage memory.

FIG. 4A illustrates a schematic diagram of a calibration device with a voltage reference circuit and a storage memory.

FIG. 4B illustrates an example of a clipped sinus signal for voltage reference.

FIG. 5 illustrates a schematic diagram of various steps performed on the audibility meter.

## Detailed description of the invention

With reference to the preceding figures, various embodiments of the device object of the invention are described.

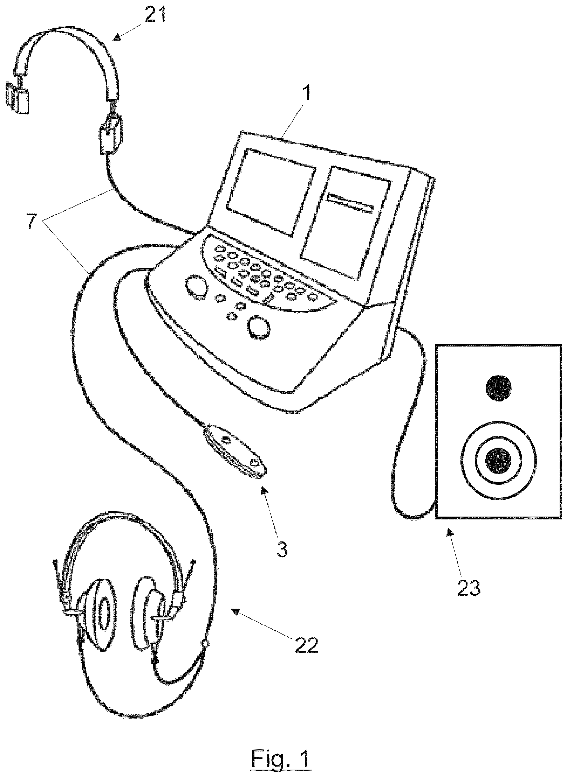

FIG. 1 shows an audiometry system that includes an audibility meter **1** connected to various types of acoustic transducers such as vibrators **21,** headphones **22** or speakers **23.** The audiometry system has a push-button **3** to record the response of the patient. The push-button **3** connects to the audibility meter **1** by means of a cable **7** with a connector adapted to be inserted into a receptacle of the audibility meter **1.** In turn, the cable connections **7** to the audibility meter **1** are made with an audio connector (e.g., audio jack or other industry standard connector) that are inserted into the also standard receptacles of the audibility meter **1.** The push-button **3** has its own memory **15** to identify the device and enable it to function as plug & play.

In operation, the audibility meter **1** produces electrical signals the features of which are controlled by a microprocessor **10** to be subsequently transformed into an acoustic signal in the transducers **21, 22, 23.** Acoustic signals, generally pure tones or voice signals, are used in the measurement of hearing thresholds, according to the accepted medical procedures. The calibration of the assembly system is divided into electrical calibration of the audibility meter **1** and acoustic calibration of the transducers **21, 22, 23.**

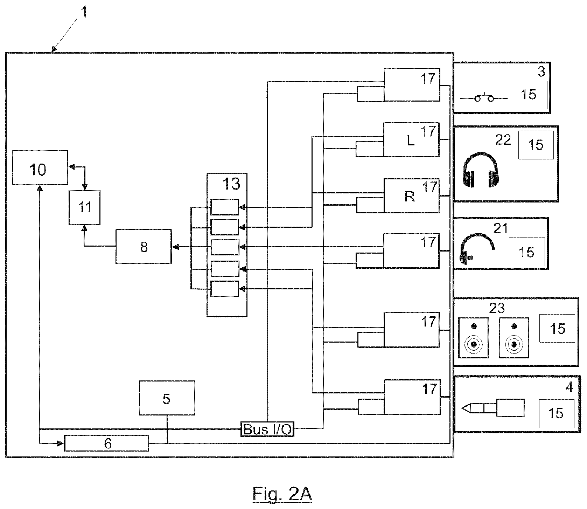

FIG. 2A is a block diagram of an embodiment of the system related to the detection of the connection of the audibility meter **1** with the possible transducers **21, 22, 23** and the reading of the calibration data thereof. It can be seen that the audibility meter **1** has a microprocessor **10** with a memory **5** and that each transducer, headphones **22,** bone vibrators **21** and speakers **23** also incorporate their own memory **15,** wherein the information regarding the individual calibration is stored. In the case of the audibility meter **1,** a reader system is incorporated **6** for these memories **15.**

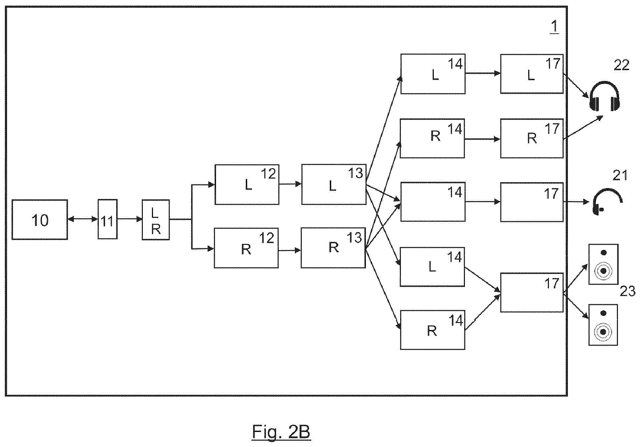

Similarly, FIG. 2B is a block diagram of another aspect of the system with the audibility meter **1** and the transducers **21, 22, 23** that illustrates how, after carrying out the electrical calibration using the external calibrator **4,** the electrical signal is internally generated in the audibility meter **1** individually, multiplexed for each R or L channel and each transducer **21, 22, 23,** so that they emit the calibrated acoustic signal.

Thus, the external calibrator **4** contains a voltage reference and a memory **15** with the device information (for plug & play) and the actual value of the voltage reference calibrated in the laboratory, in an analogous manner to the transducers **21, 22, 23.**

The reader **6** reads the information stored in the memories **15** of the transducers **21, 22, 23** (e.g., identification of the transducer, type, sensitivity data, calibration/validity date, etc.) and can compensate the recorded calibrated frequency response of the transducers **21, 22, 23,** of the audibility meter **1** itself and do it according to the frequency response of the human ear.

The adjusting algorithm implemented in the microprocessor **10** is responsible for aligning the full scale for each and every one of the working frequencies, taking into account various aspects such as the nominal full scale of the electric step of the audibility meter **1,** the nominal sensitivity of the transducer **2,** the frequency response of the human ear, the correction of the frequency response of the transducer with respect to the nominal sensitivity thereof and the correction of the frequency response of the electrical step with respect to the nominal full scale thereof. Therefore, for each working frequency of the audibility meter **1** and specific transducer **21, 22** or **23,** a correction value is obtained in dBs with respect to the nominal value of the full scale of the audibility meter **1** that must be compensated.

In more detail, the adjustment algorithm calculates the required attenuation Att[i] in dB at frequency i as follows:

$$Att[i] = FSe[i] - (FSa[i] - Sa[i]) \qquad [1]$$

FSe[i] being the electrical full scale of the audibility meter at frequency i (in dBm), FSa[i] the desired acoustic full scale taking into account the frequency response of the human ear (in dB SPL) and Sa[i] the sensitivity of the acoustic transducer in (dB SPL/dBm)

$Att[i] = FSe_n - Ee[i] - (FSa[i] - (Sn - Ea[i]))$ $FSe_n$ being is the nominal electrical full scale in dBm and Ee[i] the deviation in dB at frequency i from the nominal;

Sn being the nominal sensitivity of the transducer in dB SPL/dBm and Ea[i] the deviation of the sensitivity in dBs at frequency i with respect to the nominal one. Defining $Att_n[i] = FSe_n - FSa[i] - Sn$, $Att_n$ is called Nominal Attenuation:

$$Att[i] = Att_n[i] - Ee[i] - Ea[i] \qquad [2]$$

Specific example:
An FSa of 120dB HL (hearing level) is desired at the frequency of 1 kHz. As the ear response at 1kHz with the RadioEar DD45 type earphone is 7.5 dB SPL / dB HL, the target FSa is 127.5 dB SPL. The nominal sensitivity of the DD45 is 106.7 dB SPL / dBm. The $FSe_n$ is 31dBm. Assuming an Ee[1k] of 0.5 dB and an Ea[1k] of 2 dB

$$Attn[1k] = 31dBm - (127.5\ dB\ SPL - 106.7\ dB\ SPL/dBm) - 0.5dB - 2dB =$$
$$10.2 - 0.5 - 2\ dB = 7.7\ dB$$

Thus, the microprocessor **10** carries out said compensation at each frequency activating or deactivating attenuating devices **12**. This operating mode enables a transducer **2** with expired calibration to be replaced by a newly acoustically calibrated one avoiding a downtime of the audibility meter **1**. For remote calibration to be equivalent to regular calibration, it is also necessary to electrically calibrate the audibility meter. For this, the audibility meter **1** incorporates an electrical checking mechanism based on an analogue-digital converter **8** capable of measuring an external voltage reference and comparing it with the calibrated value stored in its own memory **5** and with the measured value of each electrical output to the transducers **21, 22, 23**. Likewise, for various working frequencies. Since the generation of electrical signals for the audibility meter **1** can be internally measured at each output and compared with the external voltage reference to know the actual value thereof, it is possible to calibrate the audibility meter system **1** automatically and electrically on site by sending a current calibrator **4**, with which it is possible to obtain the entire calibrated electrical and acoustic chain.

An audio signal generator **11** is responsible for reproducing and generating two-channel audio signals (left and right) in digital format and uses a high-quality analogue-digital converter (not shown in FIG. 2) that delivers a two-channel analogue audio signal (stereo). Currently, there are also processors that integrate their own digital audio controller that reads/writes the audio samples and sends/receives them from the DAC/ADC, so that no specific audio signal generator is required.

A digitally controlled attenuator **12** (by the microprocessor **10**) is responsible for compensating the frequency response of the transducers **21, 22, 23** and at the same time adjusting the dynamic working range of the audibility meter **1** for each frequency of use according to the information recorded in the memories **15, 5** of the transducers and the audibility meter **1 itself,** respectively.

A two-channel multiplexer (stereo) **13** is responsible for distributing the signals from the left and right channels of the generator to the output amplifiers **14** of each one of the selected transducers **21, 22 or 23** or a combination thereof.

An amplifier module **14** is responsible for amplifying the electrical signals and adapting the output to the impedance of the transducers **21, 22, 23**.

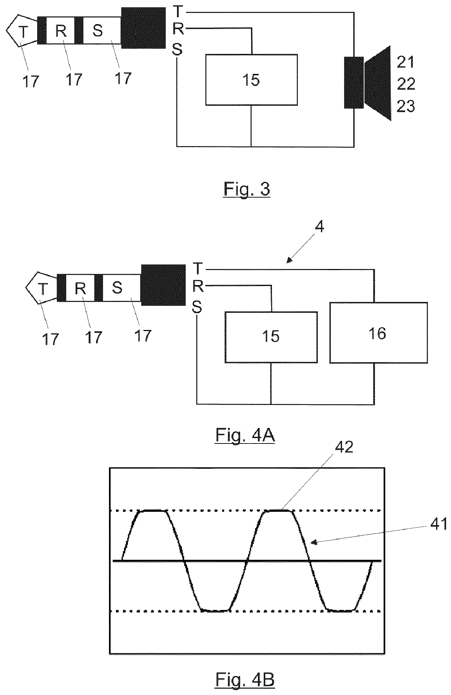

FIG. 3 illustrates a detailed schematic diagram of a transducer **21** with the memory **15 thereof** which stores useful information for calibration and connections **17** (S, T, R).

Briefly, the compensation circuitry of the frequency response of the audibility meter **1** is explained, which is generally composed of a cascade of attenuation steps by means of switched voltage dividers with analogue multiplexers and separated by voltage followers with quality audio operational amplifiers.

FIG. 4A illustrates a schematic diagram of the calibration device **4** that includes a memory **15** wherein the calibration information of the calibrator resides: (Identifier, serial number, nominal value, actual value and calibration date) and a voltage reference circuit **16** that generates a fixed value of stable electrical signal for the temperature range of use and of known and traceable value for the electrical calibration of the audibility meter **1**. The connection is also shown using standard jack connectors in audio applications, wherein between the T-S connections **17** a stable voltage is obtained provided by the voltage reference and at the connection R memory access **15** of the calibrator **4** which contains the information of the measured actual value (with traceability to standards of the international metrological system). There are jack connectors **17** that incorporate switches that can be read. Therefore, it is known in which specific connector a new transducer **21, 22, 23** or accessory has been inserted. When reading the memory **5** the transducer type and the R or L channel are read and it can be checked if the housing is wrong.

From the measurement carried out by the audibility meter calibration system, from the voltage reference with respect to the electrical signal outputs and knowing the actual value of said reference, a calibration can be carried out with knowledge of the quality thereof (uncertainty)

Similarly, when the electrical calibration of the system is required, it is sufficient to insert a voltage reference (Calibrated Voltage reference) by means of the calibration device **4** and measure it with an analogue-digital converter **8**. In this conversion step, the voltage of each one of the electrical signal outputs is also measured. From the comparison between the calibrated voltage reference read from the memory **15** thereof, the value measured by the audibility meter **1** of the voltage reference and the measurement at the signal outputs (right air conduction headphones, left air conduction headphones, bone conduction and left and right output for speakers), the signals generated by the audibility meter **1** that are injected into the transducers **21, 22, 23** can be fully verified and calibrated at an electrical level and the signal level can be readjusted so that the precision requirements necessary for the correct clinical diagnosis are fulfilled.

In order to carry out this comparison at all frequencies and all outputs for electrical calibration, one option is to modify the signals and generate clipped sinus signals using the audibility meter **1** itself at the different working frequencies.

FIG. 4B shows an example of a clipped sinus signal **41** generated by the audibility meter **1.** Measuring the plateau level **42** of this clipped sinus signal **41** and comparing it with the voltage reference, there is no need to use any other circuitry than an analogue-digital converter **8** for audio or even a comparator. Although the signal is in AC, it is used for a simple comparison in DC.

The calibration algorithm is simplified as it involves comparing the electrical level of the plateau **42** of the signal with the actual value of the voltage reference (the traceable calibration value of which is written in the memory **15** of the calibrator **4**). The outputs of the audibility meter **1** can be DC or AC coupled, and only the measurement of the voltage reference value should be DC. Thus, the audibility meter **1** does not require sophisticated or complex circuitry, as is usually necessary when using a sound level meter or the like.

Once generated in the transducer **21, 22, 23,** the acoustic signal can then be received by the patient with the intended features established by the audibility meter **1.** The measurement operation is carried out when the audibility meter **1** records a control signal actuated by the patient by means of the push-button **3** when he perceives the acoustic signal.

Previously, when the transducers **21, 22, 23** are connected to the receptacles thereof, the microprocessor **10** detects the insertion, reads the memories thereof **15** and extracts information on identification, transducer type, calibration date, sensitivity and frequency response. With all this, the audibility meter **1** can control various aspects, such as the validity of the calibration of the transducers **21, 22, 23,** the correct connection, and by means of the step of frequency compensation, it can adapt to the sensitivity, impedance and frequency response of the transducers **21, 22, 23** before emitting acoustic stimuli to the patient.

Finally, more details of a preferred embodiment are provided, electronic cards are incorporated into the transducers **21, 22, 23,** on the push-button **3** and in the calibrator **4,** series-reading memories **15** are included that in this case use Maxim™ (formerly Dallas) technology for "one-wire" devices, specifically the DS28E05 memory, although they can be similar devices, or from other manufacturers (whether or not they require self-powering circuits). The "one-wire" technology enables the memories to be integrated into the standard audio connectors common in audibility meters **1** and in soundproofed audiometry booths without the need to add self-powered circuits or to extract energy from the audio signals by connecting to the central contact of the audio connector (R-Ring). The information of the electronic cards that resides in the series-type memories **15** is read by the microprocessor **10** via the serial bus ("one-wire" in the case of Maxim). For adjusting calibration and maintenance, the transducers **21, 22, 23** (headphones, high frequency headphones, insert earphones, speakers and bone vibrator) are provided with an electronic card (similar to TEDS Transducer Electronic Data Sheet (IEEE 1451.4)) integrated in the connector with a memory **15** containing at least data related to:

- Identification and serial number of the transducer.
- Type of transducer.
- Left/Right Channel
- Sensitivity and frequency response thereof.
- Date of calibration.

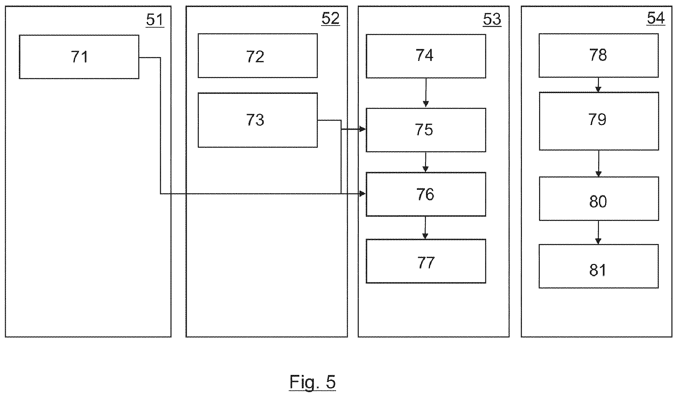

FIG. 5 schematically shows a flow diagram of four processes that can be asynchronous with several steps performed by the microprocessor of the audibility meter. It starts with an initial process **51** with one step **71** of reading the audibility meter's own memory when starting the audibility meter. There is then a second connection process **52** with the transducer that is triggered when a transducer connection is detected in the step **72** of detecting connection, which is followed by a second step **73** of reading the memory of the transducer wherein it is verified that the connection is correct. When a working frequency and a transducer are selected, the adjustment process is carried out with the information of the memories read in the steps **71** and **73,** and the adjustment algorithm already explained and summarised in formulas [1] and [2] is applied. Basically, it is a matter of setting the maximum audio generation value for each one of the frequencies. This third adjustment process **53** includes a first step **74 of** reading the acoustic full scale parameter (FSa) in dBHL for that frequency and transducer, a second step **75** of calculating the acoustic full scale (FSa) in dB SPL or dBuN, taking into account the frequency response of the human ear; subsequently, a third step **76** of calculating the attenuation value (Att) at that frequency and transducer is carried out; and ends with a fourth step **77** of selecting the value of the attenuation step. This attenuation value is static as long as the operating frequency or transducer are not changed.

Therefore, with the above information, a calibrated audio signal can be obtained in the transducer and a correct hearing level measurement can be carried out. Thus, when the fourth process **54** of generating audio at a certain level is run the following are carried out: a first step **78** of selecting the specific type of signal to be reproduced in the audiometry test, then in a second step **79** of selecting the output route, acting on the multiplexer. It continues with a third step **80** of establishing the output level in the DAC converter to finally in a fourth step **81** of emitting audio signal, reproduce the calibrated audio signal with a determined intensity and frequency on the selected acoustic transducer.

Thanks to the above advantages, it is possible to maintain the metrological veracity of the audibility meter **1** carrying out an adjustment of the electrical calibration remotely and replacing the transducers **2** with an expired calibration period by others with current calibration, simplifying, speeding up and lowering costs of the start-up and maintenance thereof.

In conclusion, the audibility meter system described in this invention automatically solves the adjustment of the acoustic calibration of any transducer **21, 22, 23** and the electrical calibration of the audibility meter itself **1** maintaining the metrological veracity necessary to carry out the clinical diagnosis with guarantees.

NUMERICAL REFERENCES

1 Audibility meter.
3 Push-button.
4 Calibrator.
5 Microprocessor memory.
6 Memory reader.
8 Analogue-digital converter.
11 Audio/digital/electrical signal generator.
12 Digital attenuator.
13 Multiplexer.
14 Amplifier.
15 Transducer/calibrator/push-button memory
17 TRS connection
21 Vibrator (transducer).
22 Headphone (transducer).
23 Speaker (transducer).
41 Clipped sinus signal
42 Plateau
51 initial process
52 process of connecting with the transducer
53 adjustment process
54 process of generating audio
71 step of reading the memory of the audibility meter
72 step of detecting connection
73 step of reading the memory of the transducer
74 step of reading the acoustic full scale parameter
75 step of calculating the acoustic full scale
76 step of calculating the attenuation value
78 step of selecting the signal type
79 step of selecting the output route
80 step of establishing the output level
81 step of emitting audio signal

**Claims**

1. An audibility meter calibration system comprising:

    - an acoustic transducer (21,22,23) configured to produce an acoustic signal from an electrical signal, wherein the acoustic transducer (21,22,23) comprises a memory (15), wherein identification information and calibration information of said transducer (21,22,23) are stored;
    - an audibility meter (1) configured to generate an electrical signal capable of producing a corresponding acoustic signal in the transducer (21,22,23), wherein the audibility meter (1) comprises a microprocessor (10), and a memory (5) wherein calibration information of the audibility meter itself (1) is stored and
    wherein the audibility meter (1) is also configured to read the memory (15) of the acoustic transducer (21,22,23) and to compensate the response of the acoustic transducer (21,22,23) based on the calibration information read on the memory (15) of the acoustic transducer (21,22,23) and on the frequency response of the human ear; **characterised in that** the audibility meter calibration system further comprises:

- a calibration device (4) comprising a voltage reference circuit (16) configured to generate at least one voltage reference with a specific value in the audibility meter (1) to which it is connected;

and **in that** the audibility meter (1) is further configured to compensate the frequency response of the audibility meter itself (1) by modifying the electrical signal generated, and to update the calibration information for same based on the voltage reference for the calibration device (4).

2. The audibility meter calibration system according to claim 1, wherein the microprocessor (10) of the audibility meter (1) is configured to compare the calibration information of the acoustic transducer (21,22,23) with that of the audibility meter itself (1) stored in the memory (5) thereof.

3. The audibility meter calibration system according to any one of claim 1 or 2, wherein the microprocessor (10) is configured to align the full scale of the audibility meter (1) with the nominal full scale for a plurality of working frequencies.

4. The audibility meter calibration system according to any one of claims 1 to 3, wherein the audibility meter (1) comprises an analogue-digital converter (8) for measuring an external voltage reference provided by the calibration device (4).

5. The audibility meter calibration system according to any one of claims 1 to 4, wherein the voltage reference circuit (16) of the calibration device (4) is configured to generate a plurality of calibrated sinus and/or broadband signals associated with a plurality of working and/or broadband frequencies of the audibility meter (1).

6. The audibility meter calibration system according to any one of claims 1 to 5, wherein the audibility meter (1) comprises a digital attenuator (12) and a multiplexer (13).

7. The audibility meter calibration system according to any one of claims 1 to 6, wherein the calibration information of said transducer (21,22,23) comprises at least one of the following data: identification of the transducer, serial number, type of transducer, sensitivity, the frequency response thereof and calibration date.

8. The audibility meter calibration system according to any one of claims 1 to 7, wherein the audibility meter (1) is configured to generate, for one or more frequencies, a clipped sinus signal (41), comparing the amplitude of said clipped signal with the voltage reference of the calibration device (4) written in a memory (15) of the calibration device (4).

**Patentansprüche**

1. Kalibriersystem für Hörbarkeitsmessgerät umfassend:

- einen akustischen Wandler (21, 22, 23), welcher dazu ausgebildet ist, ein akustisches Signal aus einem elektrischen Signal zu produzieren, wobei der akustische Wandler (21, 22, 23) einen Speicher (15) umfasst, in welchem Identifizierungsinformation und Kalibrierinformation des genannten Wandlers (21, 22, 23) gespeichert werden;
- ein Hörbarkeitsmessgerät (1), welches dazu ausgebildet ist, ein elektrisches Signal zu erzeugen, welches in der Lage ist, ein entsprechendes akustisches Signal im Wandler (21, 22, 23) zu produzieren, wobei das Hörbarkeitsmessgerät (1) einen Mikroprozessor (10) und einen Speicher (5), in welchem Kalibrierinformation des Hörbarkeitsmessgeräts selbst (1) gespeichert ist, umfasst und wobei das Hörbarkeitsmessgerät (1) auch dazu ausgebildet ist, den Speicher (15) des akustischen Wandlers (21, 22, 23) zu lesen und die Antwort des akustischen Wandlers (21, 22, 23) basierend auf der im Speicher (15) des akustischen Wandlers (21, 22, 23) gelesenen Kalibrierinformation und auf der Frequenzantwort des menschlichen Ohrs zu kompensieren; **dadurch gekennzeichnet, dass** das Kalibriersystem für Hörbarkeitsmessgerät zusätzlich Folgendes umfasst:

- eine Kalibriervorrichtung (4), welche einen Spannungsreferenzkreis (16) umfasst, welcher dazu ausgebildet ist, mindestens eine Spannungsreferenz mit einem spezifischen Wert im Hörbarkeitsmessgerät (1), mit welchem er verbunden ist, zu erzeugen;

und dass das Hörbarkeitsmessgerät (1) zusätzlich dazu ausgebildet ist, die Frequenzantwort des Hörbarkeitsmessgeräts selbst (1) zu kompensieren, indem das erzeugte elektrische Signal modifiziert wird, und die Kalibrierinformation für dasselbe basierend auf der Spannungsreferenz für die Kalibriervorrichtung (4) zu aktualisieren.

2. Kalibriersystem für Hörbarkeitsmessgerät nach Anspruch 1, wobei der Mikroprozessor (10) des Hörbarkeitsmessgeräts (1) dazu ausgebildet ist, die Kalibrierinformation des akustischen Wandlers (21, 22, 23) mit derjenigen des Hörbarkeitsmessgeräts selbst (1), welche im Speicher (5) desselben gespeichert ist, zu vergleichen.

3. Kalibriersystem für Hörbarkeitsmessgerät nach einem von Anspruch 1 oder 2, wobei der Mikroprozessor (10) dazu ausgebildet ist, den Skalenendwert des Hörbarkeitsmessgeräts (1) mit dem nominalen Skalenendwert für eine Vielzahl von Arbeitsfrequenzen auszurichten.

4. Kalibriersystem für Hörbarkeitsmessgerät nach einem der Ansprüche 1 bis 3, wobei das Hörbarkeitsmessgerät (1) einen Analog-Digital-Umsetzer (8) zum Messen einer äußeren Spannungsreferenz, welche von der Kalibriervorrichtung (4) bereitgestellt wird, umfasst.

5. Kalibriersystem für Hörbarkeitsmessgerät nach einem der Ansprüche 1 bis 4, wobei der Spannungsreferenzkreis (16) der Kalibriervorrichtung (4) dazu ausgebildet ist, eine Vielzahl von kalibrierten Sinus- und/oder Breitbandsignalen zu erzeugen, welche mit einer Vielzahl von Arbeits- und/oder Breitbandfrequenzen des Hörbarkeitsmessgeräts (1) assoziiert sind.

6. Kalibriersystem für Hörbarkeitsmessgerät nach einem der Ansprüche 1 bis 5, wobei das Hörbarkeitsmessgerät (1) ein digitales Dämpfungsglied (12) und einen Multiplexer (13) umfasst.

7. Kalibriersystem für Hörbarkeitsmessgerät nach einem der Ansprüche 1 bis 6, wobei die Kalibrierinformation des genannten Wandlers (21, 22, 23) mindestens eines der folgenden Daten umfasst: Identifizierung des Wandlers, Seriennummer, Art des Wandlers, Empfindlichkeit, die Frequenzantwort desselben und Kalibrierdatum.

8. Kalibriersystem für Hörbarkeitsmessgerät nach einem der Ansprüche 1 bis 7, wobei das Hörbarkeitsmessgerät (1) dazu ausgebildet ist, für eine oder mehrere Frequenzen, ein übersteuertes Sinussignal (41) zu erzeugen, wobei die Amplitude des genannten übersteuerten Signals mit der Spannungsreferenz der Kalibriervorrichtung (4), welche in einem Speicher (15) der Kalibriervorrichtung (4) geschrieben ist, verglichen wird.

**Revendications**

1. Système d'étalonnage d'un audiomètre, comprenant

- un transducteur acoustique (21, 22, 23) configuré pour produire un signal acoustique à partir d'un signal électrique, dans lequel le transducteur acoustique (21, 22, 23) comprend une mémoire (15), dans laquelle des informations d'identification et des informations d'étalonnage dudit transducteur (21, 22, 23) sont stockées ;
- un audiomètre (1) configuré pour générer un signal électrique capable de produire un signal acoustique correspondant dans le transducteur (21, 22, 23), dans lequel l'audiomètre (1) comprend un microprocesseur (10), et une mémoire (5) dans laquelle des informations d'étalonnage de l'audiomètre lui-même (1) sont stockées, et

dans lequel l'audiomètre (1) est également configuré pour lire la mémoire (15) du transducteur acoustique (21, 22, 23) et pour compenser la réponse du transducteur acoustique (21, 22, 23) sur la base des informations d'étalonnage lues sur la mémoire (15) du transducteur acoustique (21, 22, 23) et sur la réponse en fréquence de l'oreille humaine ;
**caractérisé en ce que** le système d'étalonnage de l'appareil de mesure de l'audibilité comprend en outre ;

- un dispositif d'étalonnage (4) comprenant un circuit de référence de tension (16) configuré pour générer au moins une référence de tension avec une valeur spécifique dans l'audiomètre (1) auquel il est connecté ;

et **en ce que** l'audiomètre (1) est en outre configuré pour compenser la réponse en fréquence du compteur d'audibilité lui-même (1) en modifiant le signal électrique généré, et pour mettre à jour les informations d'étalonnage de celui-ci sur la base de la référence de tension pour le dispositif d'étalonnage (4).

2. Système d'étalonnage d'un audiomètre selon la revendication 1, dans lequel le microprocesseur (10) de l'audiomètre (1) est configuré pour comparer les informations d'étalonnage du transducteur acoustique (21, 22, 23) avec celles de l'audiomètre lui-même (1) stockées dans sa mémoire (5).

3. Système d'étalonnage d'un audiomètre selon l'une quelconque des revendications 1 ou 2, dans lequel le microprocesseur (10) est configuré pour aligner la pleine échelle de l'audiomètre (1) avec la pleine échelle nominale pour une pluralité de fréquences de travail.

4. Système d'étalonnage de compteur d'audibilité selon l'une quelconque des revendications 1 à 3, dans lequel l'audiomètre (1) comprend un convertisseur analogique-numérique (8) pour mesurer une référence de tension externe fournie par le dispositif d'étalonnage (4).

5. Système d'étalonnage d'un audiomètre selon l'une quelconque des revendications 1 à 4, dans lequel le circuit de référence de tension (16) du dispositif d'étalonnage (4) est configuré pour générer une pluralité de signaux sinus et/ou large bande étalonnés associés à une pluralité de fréquences de travail et/ou large bande de l'audiomètre (1).

6. Système d'étalonnage d'un audiomètre selon l'une quelconque des revendications 1 à 5, dans lequel l'audiomètre (1) comprend un atténuateur numérique (12) et un multiplexeur (13).

7. Système d'étalonnage d'un audiomètre selon l'une quelconque des revendications 1 à 6, dans lequel les informations d'étalonnage dudit transducteur (21, 22, 23) comprennent au moins l'une des données suivantes : identification du transducteur, numéro de série, type de transducteur, sensibilité, réponse en fréquence de celui-ci et date d'étalonnage.

8. Système d'étalonnage d'un audiomètre selon l'une quelconque des revendications 1 à 7, dans lequel l'audiomètre (1) est configuré pour générer, pour une ou plusieurs fréquences, un signal sinusoïdal écrêté (41), en comparant l'amplitude dudit signal écrêté avec la référence de tension du dispositif d'étalonnage (4) inscrite dans une mémoire (15) du dispositif d'étalonnage (4).

Fig. 1

Fig. 2A

Fig. 2B

Fig. 3

Fig. 4A

Fig. 4B

Fig. 5

**REFERENCES CITED IN THE DESCRIPTION**

**Patent documents cited in the description**

- EP 1854407 A1

- US 20110009770 A1